# EUROPEAN PATENT APPLICATION

(11) **EP 4 241 665 A1**
(43) Date of publication of application: **13.09.2023**
(21) Application number: 23155998.0
(22) Date of filing: 10.02.2023
(51) Int. Cl.: A61B 5/0215, A61B 5/00, A61F 2/07

(54) **ENDOVASCULAR PRESSURE GRADIENT DETERMINATION METHOD**

(30) Priority: 08.03.2022 US 202217689475
(71) Applicant: Medtronic Vascular, Inc., Santa Rosa, CA 95403 (US)
(72) Inventor: PERKINS, Keith, Santa Rosa 95403 (US); TRICARICO, Rosamaria, Santa Rosa 95403 (US); STIGER, Mark L., Santa Rosa 95403 (US)
(74) Representative: Zimmermann & Partner Patentanwälte mbB

(57) **Abstract**

A method for determining a pressure gradient between proximal and distal locations within a vasculature of a patient. The method includes deploying an ancillary device within the vasculature. The ancillary device integrates a proximal sensor at the proximal location and a distal sensor at the distal location. The method further includes deploying, via a stent graft delivery system, a stent graft within the vasculature to obtain a deployed stent graft. The ancillary device is independent from the stent graft delivery system and the stent graft. The method further includes outputting proximal pressure data from the proximal sensor and indicative of pressure at the proximal location and distal pressure data from the distal sensor and indicative of pressure at the distal location. The method also includes determining the pressure gradient using the proximal and distal pressure data.

## Description

### TECHNICAL FIELD

The present disclosure relates generally to an endovascular pressure gradient determination method. The method may determine a pressure gradient between proximal and distal locations within a vasculature of a patient. The determined pressure gradients may be used to detect the presence or absence of endoleaks or a continued false lumen pressurization state.

### BACKGROUND

An endovascular repair procedure may be used to repair an abnormality or lesion (e.g., an aneurysm, a false lumen, or a dissection), at a vascular site (e.g., a blood vessel or other body passageways) in the vascular system of a patient. The endovascular repair procedure may exclude the abnormality from blood flowing through the vascular system of the patient, thereby depressurizing the abnormality to mitigate potential negative consequences (e.g., rupture) of the abnormality.

An endovascular repair procedure may be carried out using an expandable stent graft. The stent graft has a surgical graft covering and an expanding (e.g., self-expanding) metal frame. The stent graft may be positioned at a target vascular site in a radially compressed state and subsequently expanded to a radially expanded state to bridge the abnormality to restore the function of the vascular site damaged by the abnormality.

### SUMMARY

According to one embodiment, a method for determining a pressure gradient between proximal and distal locations within a vasculature of a patient is disclosed. The method includes deploying an ancillary device within the vasculature. The ancillary device integrates a proximal sensor at the proximal location and a distal sensor at the distal location. The method further includes deploying, via a stent graft delivery system, a stent graft within the vasculature to obtain a deployed stent graft. The ancillary device is independent from the stent graft delivery system and the stent graft. The method further includes outputting proximal pressure data from the proximal sensor and indicative of pressure at the proximal location and distal pressure data from the distal sensor and indicative of pressure at the distal location. The method also includes determining the pressure gradient using the proximal and distal pressure data.According to another embodiment, a method for determining a pressure gradient between proximal and distal locations within a vasculature of a patient is disclosed. The method includes deploying a guidewire within the vasculature. The method further includes deploying first and second nested catheters over the guidewire within the vasculature. The first nested catheter integrates a distal sensor at the distal location. The second nested catheter integrates a proximal sensor at the proximal location. The method also includes deploying a stent graft within the vasculature to obtain a deployed stent graft. The method further includes outputting proximal pressure data from the proximal sensor and indicative of pressure at the proximal location and distal pressure data from the distal sensor and indicative of pressure at the distal location. The method also includes determining the pressure gradient using the proximal and distal pressure data.

According to yet another embodiment, an endovascular pressure sensing device for outputting pressure data indicative of a pressure gradient between proximal and distal locations within a vasculature of a patient is disclosed. The endovascular pressure sensing device includes an ancillary device deployable within the vasculature relative to a stent graft deployable within the vasculature. The ancillary device independent from the stent graft. The endovascular pressure sensing device further includes proximal and distal sensors. The ancillary device is configured to integrate the proximal sensor at the proximal location and the distal sensor at the distal location. The proximal sensor is configured to output proximal pressure data indicative of pressure at the proximal location and the distal sensor is configured to output distal pressure data indicative of pressure at the distal location.

Further disclosed herein is a method for determining a pressure gradient between proximal and distal locations within a vasculature of a patient, wherein the method includes deploying an ancillary device within the vasculature, wherein the ancillary device integrates a proximal sensor at the proximal location and a distal sensor at the distal location, wherein the method further includes deploying, via a stent graft delivery system, a stent graft within the vasculature to obtain a deployed stent graft, wherein the ancillary device is independent from the stent graft delivery system and the stent graft, wherein the method further includes outputting proximal pressure data from the proximal sensor and indicative of pressure at the proximal location and distal pressure data from the distal sensor and indicative of pressure at the distal location, wherein the method also includes determining the pressure gradient using the proximal and distal pressure data.

Further disclosed herein are systems configured for performing the above-described methods.

### BRIEF DESCRIPTION OF THE DRAWINGS

Figure 1A is a side view of a stent graft in a radially compressed state and an ancillary device within an aorta of a patient during deployment of the stent graft within the aorta.
Figure 1B is a side view of the stent graft of Figure 1A in a radially expanded state and deployed within the aorta with the ancillary device contralateral the stent graft.
Figure 2 is a side view of a deployed stent graft in a radially expanded state and an ancillary device introducing pressure reducing agents into an abnormality cavity of the aorta.
Figure 3 is a side view of a deployed stent graft in a radially expanded state and an ancillary device partially extending through a portion of the deployed stent graft.
Figure 4A is a side view of an ancillary device including first and second ancillary device components.
Figure 4B is a side view of an ancillary device including a guidewire and first, second, and third nested catheters movable relative each other and the guidewire.
Figure 5 is a side view of an ancillary device delivery system.
Figure 6 is a side view of an ancillary imaging device delivery system.

### DETAILED DESCRIPTION

Embodiments of the present disclosure are described herein. It is to be understood, however, that the disclosed embodiments are merely examples and other embodiments can take various and alternative forms. The figures are not necessarily to scale; some features could be exaggerated or minimized to show details of particular components. Therefore, specific structural and functional details disclosed herein are not to be interpreted as limiting, but merely as a representative basis for teaching one skilled in the art to variously employ the embodiments. As those of ordinary skill in the art will understand, various features illustrated and described with reference to any one of the figures can be combined with features illustrated in one or more other figures to produce embodiments that are not explicitly illustrated or described. The combinations of features illustrated provide representative embodiments for typical applications. Various combinations and modifications of the features consistent with the teachings of this disclosure, however, could be desired for particular applications or implementations.

Directional terms used herein are made with reference to the views and orientations shown in the exemplary figures. A central axis is shown in the figures and described below. Terms such as "outer" and "inner" are relative to the central axis. For example, an "outer" surface means that the surfaces faces away from the central axis, or is outboard of another "inner" surface. Terms such as "radial," "diameter," "circumference," etc. also are relative to the central axis. The terms "front," "rear," "upper" and "lower" designate directions in the drawings to which reference is made.

Unless otherwise indicated, for the delivery system the terms "distal" and "proximal" are used in the following description with respect to a position or direction relative to a treating clinician. "Distal" and "distally" are positions distant from or in a direction away from the clinician, and "proximal" and "proximally" are positions near or in a direction toward the clinician. For the stent-graft prosthesis, "proximal" is the portion nearer the heart by way of blood flow path while "distal" is the portion of the stent-graft further from the heart by way of blood flow path.

The following detailed description is merely exemplary in nature and is not intended to limit the invention or the application and uses of the invention. Although the description is in the context of treatment of blood vessels such as the aorta, coronary, carotid, and renal arteries, the invention may also be used in any other body passageways (e.g., aortic valves, heart ventricles, and heart walls) where it is deemed useful. For example, one or more embodiments may be useful in peripheral applications to determine an intervention has reestablished adequate flow. As another example, one or more embodiments may be useful to determine that an intervention to correct an aortic coarctation was sufficient.

A patient may experience a complication during or after an endovascular repair procedure to repair an abnormality or lesion (e.g., an aneurysm, a false lumen, or a dissection). Non-limiting examples of complications that typically occur soon after the procedure include endoleaks and stent graft-induced new entry tear (SINE). An endoleak occurs when some amount of blood flow remains in the abnormality after the endovascular repair procedure using a stent graft. SINE occurs when the stent graft induces a tear at the target vascular site during the endovascular repair procedure. Continued blood flow to an endoleak or a SINE causes elevation of pressure within an aneurysm sac or false lumen. Under these circumstances, the endovascular repair procedure fails to reduce pressure within the aneurysm sac or false lumen.

These complications during endovascular repair procedures may be difficult to detect at the completion of the procedure. Two-dimensional (2D) image projections from fluoroscopic imaging may not be able to discern endoleaks in a complex three-dimensional (3D) anatomy. Occult endoleaks may not be evident during the primary endovascular repair procedure when the patient is in a prone position and blood pressure is controlled. Certain graft materials (e.g., polyethylene terephthalate (PET)) are semipermeable and are configured to become impermeable as blood clots form in the semipermeable graft material. Trans graft perfusate during this process may confound the detection of endoleaks.

The inability to adequately detect these complications at the end of the endovascular repair procedure makes it difficult to confirm these complications are not present. For this reason, a patient may need to return for an early invasive and costly computed tomography (CT) scan to confirm that such pathology is excluded. In a large percentage of cases where these complications exist, a later reintervention is used to address the complication. The reintervention is a second, invasive procedure that may be avoided if the pathology is properly identified during or soon after the initial procedure so that it can be remedied during the initial procedure. What is needed is a process to definitively determine during or soon after the index procedure that the abnormality was successfully excluded from a patient's vessel circulation. A definitive determination during or soon after the index procedure may permit immediate adjunctive intervention, thereby reducing secondary procedures and lowering the burden for costly invasive follow-up.

One or more embodiments disclose a plurality of pressure sensors (e.g., transducers) configured to simultaneously track systemic and abnormality cavity pressure periprocedurally. One or more embodiments provide a noninvasive, inexpensive mechanism to track changes in abnormality cavity pressure (e.g., aneurysmal sac) relative to systemic pressure. These measurements may occur in real time using ancillary devices deployed for other purposes. One or more embodiments include a controller programmed to determine waveforms from pressure data output by the sensors and format the pressure waveforms superimposed side by side to permit a direct comparison of output from each of the sensors.

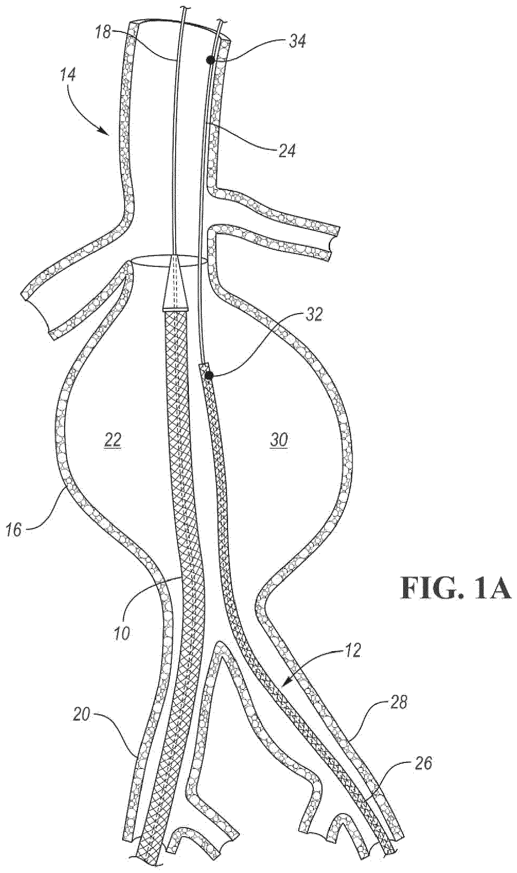

Figure 1A is a side view of stent graft 10 in a radially compressed state and ancillary device 12 within aorta 14 of a patient during deployment of stent graft 10 within aorta 14. While stent graft 10 and ancillary device 12 are shown within aorta 14, these devices may be applied to other vasculature (e.g., blood vessels) within a patient's body. As shown in Figure 1A, stent graft 10 is being deployed within aneurysm 16, which is a non-limiting example of an abnormality within a patient's vasculature. Stent graft 10 in the radially compressed state tracks along guidewire 18 through first iliac artery 20 to ipsilateral access site 22. Ancillary device 12 includes guidewire 24 and catheter 26. Catheter 26 tracks along guidewire 24 through second iliac artery 28 to contralateral access site 30. Ancillary device 12 is situated outside of stent graft 10. In one embodiment, ancillary device 12 (e.g., guidewire 24) is deployed before guidewire 18 is deployed for stent graft 12.

Ancillary device 12 includes distal and proximal pressure sensors 32 and 34. Distal pressure sensor 32 is integrated into catheter 26 in a distal portion thereof. Proximal pressure sensor 34 is integrated into guidewire 24. The locations of distal and/or proximal pressure sensors 32 and 34 on guidewire 24 and/or catheter 26, respectively, may be adjusted provided that a distance is maintained between distal and proximal sensors 32 and 34 so that pressure data output from the sensors may be used to determine a pressure gradient between the locations. In one or more embodiments, the pressure sensor integrated into catheter 26 may be the proximal sensor and the pressure sensor integrated into guidewire 24 may be the distal sensor.

Ancillary device 12 may be a device that is independent from the functioning and structure of stent graft 10. An ancillary device may conventionally perform a different function (e.g., imaging catheter) than outputting pressure data from one or more sensors. For instance, wires and imaging catheters are placed in an aneurysm sac as a typical part of a procedure without negatively impacting device performance. Accordingly, in one or more embodiments, the pressure sensing function and structure may be further integrated into an existing ancillary device with lower incremental time and costs than to develop a special purpose device for outputting pressure data. Distal and proximal pressure sensors 32 and 34 may alternatively be tracked into a target vascular site via supra-aortic access depending on the endovascular procedure.

In one or more embodiments, one or more pressure sensors (e.g., distal and proximal pressure sensors 32 and 34) may be a pressure transducer, a solid-state sensor, or a minimally invasive monitoring sensor (MIMS). The one or more pressure sensors may be electrically coupled to a controller via electrical connectors (e.g., conductive wires). The one or more pressure sensors may be wirelessly connected to the electrical connectors using active or passive telemetry. The controller may be configured to receive the output from the sensors. For example, proximal pressure sensor 34 may be configured to output proximal pressure data indicative of pressure over time at the proximal location and distal pressure sensor 32 may be configured to output proximal pressure data indicative of pressure over time at the distal location. The controller may be programmed to receive the proximal and distal pressure data. The controller may be further programmed to determine a pressure gradient, either instantaneously or over time, between the proximal and distal locations within the vasculature of the patient in response to the proximal and distal pressure data. The controller may also be programmed to format the pressure gradient for display on a monitor.

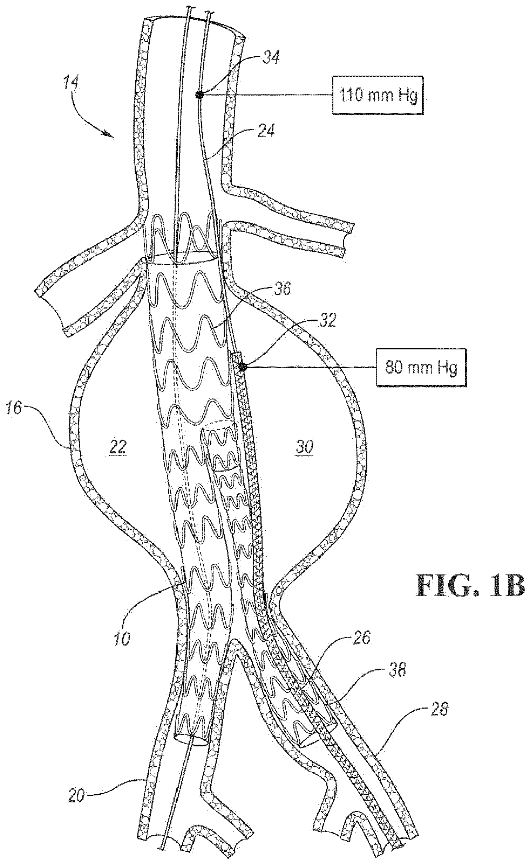

Figure 1B is a side view of stent graft 10 in a radially expanded state such that stent graft 10 is deployed (e.g., referred to as an index procedure) within aneurysm 16 to bridge a damaged portion of aorta 14. As shown in Figure 1B, stent graft 10 is a branched stent graft including main body 36 extending into first iliac artery 20 and leg 38 extending into second iliac artery 28. In other embodiments, the stent graft may only include a main body without branching or may have additional or different branches.

An objective of deploying stent graft 10 within aneurysm 16 is to exclude aneurysm 16 from blood flow, thereby depressurizing to mitigate potential negative consequences of aneurysm 16. Ancillary device 12 integrating distal and proximal pressure sensors 32 and 34 may be used to determine whether the deployment stent graft 10 reduced pressure adequately within aneurysm 16 during a monitoring period relative to a completion of the deployment of stent graft 10. For instance, the monitoring period may begin prior to deployment or upon the completion of the deployment and end after an adequate amount of time passes to determine whether the endovascular procedure was successful. The monitoring period may be any of the following values or in a range of any two of the following values: 1, 2, 3, 4, 5, 6, 7, or 8 minutes. In one embodiment, the monitoring period (e.g., pressure tracking) begins just prior to graft deployment. At this point in time, readings from the proximal pressure sensor 34 (e.g., systemic pressure sensor) should match distal pressure sensor 32 (e.g., sac pressure sensor). Upon completion of the deployment of stent graft 10, a successful deployment should demonstrate a decrease in sac pressure over the next 2 to 3 minutes. If no pressure gradient develops that may alert the physician to the possible presence of an endoleak. In response, the physician may inflate a balloon at the proximal and/or distal landing zones to attempt to develop a pressure gradient, thereby indicating type 1 or type 1b endoleaks. If the pressure gradient exists when the balloon (of the proximal and/or distal landing zones) is inflated but resolves when the balloon is deflated, the physician may consider adding one or more endoanchors at the deployment site and/or extending the proximal or distal landing zones. If no pressure change occurs in sac pressure when the balloon is inflated, then the junctional overlap may be ballooned to rule out a type 3 endoleak. If that also has no impact on developing a pressure gradient, then the physician may interrogate the region for a type 2 endoloeak. If a type 2 endoleak is detected, then the physician may consider deploying coils or embolic materials. In one embodiment, these actions may be carried out during the monitoring period.

The magnitude of the presence of a gradient may also provide support to identify the type of endoleak during the monitoring period. Relatively high flow leaks (e.g., type 1 or type 3 endoleaks) would likely prevent the development of any gradient while a relatively small gradient would indicate a low flow leak (e.g., type 2 endoleak). Changes in the pressure waveform may also provide support to identify the type of endoleak. The type of graft material may also impact the speed at which a gradient develops (e.g., during the monitoring period). Expanded polytetrafluoroethylene (ePTFE) may result in a rapid decrease (e.g., 30 seconds to 2 minutes) while PET may take several minutes (e.g., 2 to 10 minutes) to clot off.

Once stent graft 10 is completely deployed, the location of distal pressure sensor 32 may be adjusted by moving catheter 26 relative to guidewire 24 to conveniently locate distal pressure sensor 32 within the sac of the aneurysm 16 such that distal pressure sensor 32 detects and outputs pressure data indicative of pressure over time within the sac of the aneurysm. This step provides the feature of permitting movement of one pressure sensor relative to another pressure sensor to change the distance between the two sensors to obtain useful data regarding the potential presence of endoleaks (e.g., at different locations within the abnormality). This location information may also aid the physician in determining the type of endoleak and be useful in developing a mitigation strategy. After deployment, the location of proximal pressure sensor 34 may be proximal the proximal end of stent graft 10 so that proximal pressure sensor 34 detects and outputs pressure data indicative of unperturbed systematic blood pressure instantaneously or over time. While Figure 1B depicts aneurysm 16 as the abnormality and the sac as the abnormality cavity, distal and proximal pressure sensors 32 and 34 may be applied to other vascular abnormalities.

The ancillary device may be situated outside the deployed stent graft while the outputting step is performed to not interfere with the deployment of the stent graft. Distal and/or proximal pressure sensors 32 and 34 may continuously detect and output pressure data. Distal and proximal pressure sensors 32 and 34 may simultaneously detect and output pressure data to facilitate monitoring a comparison of sac pressure versus systematic pressure in real time after completion of the endovascular repair procedure. This may allow for a pressure gradient between the two locations to be determined and displayed (e.g., systolic aneurysm sac pressure and systolic aortic pressure as shown in the boxes on Figure 1B after stent graft opening, or diastolic aneurysm sac pressure and diastolic aortic pressure), either continuously, as regular intervals, or at a single point in time (e.g., when requested by a physician, such as by pushing a button, pulling a trigger, etc.).

The target pressure gradient may depend in part of the type of graft material and may be impacted by the location of the vasculature. The pressure gradient may further have variability based on the presence of endoleaks and patient specific factors like antiplatelet therapy. The target pressure gradient may be any of the following values or in a range of any two of the following values: 20, 21, 22, 23, 24, 25, 26, 27, 28, 29, and 30%. However, the development of this target pressure gradient may not be immediate and may take time to develop. In one or more embodiments, a threshold pressure gradient of 5, 6, 7, 8, 9, 10, 11, 12, 13, 14, 15, 16, 17, 18, 19, or 20% (or any range of two of these values) may form during the monitoring period to signal an endovascular repair procedure without an endoleak.

In the minutes following deployment of stent graft 10 (e.g., the monitoring period), an endovascular repair procedure without an endoleak should exhibit an attenuation in blood pressure of the aneurysm sac relative to the systemic blood pressure (e.g., a pressure gradient), and a dampening of the pressure waveform in the aneurysm sac relative to the systemic blood pressure. Relative to Figure 1B, a type of endoleak that may form is a type 1 endoleak where the leak forms directly between the aneurysm sac and the stent graft. The blood pressure gradient as a percentage difference between a blood pressure of the abnormality and the system blood pressure demonstrating the absence of endoleaks may be at least any of the following values or in a range of any two of the following values: 5, 6, 7, 8, 9, and 10%. If a percentage difference in the pressure gradient fails to develop, it may indicate that the aneurysm has not been successfully excluded from the parent circulation and/or the aneurysm has not been adequately depressurized. Such a finding during the monitoring period immediately after the completion of the deployment permits an implanting physician to intervene immediately to remedy the endoleak without a second procedure later in time.

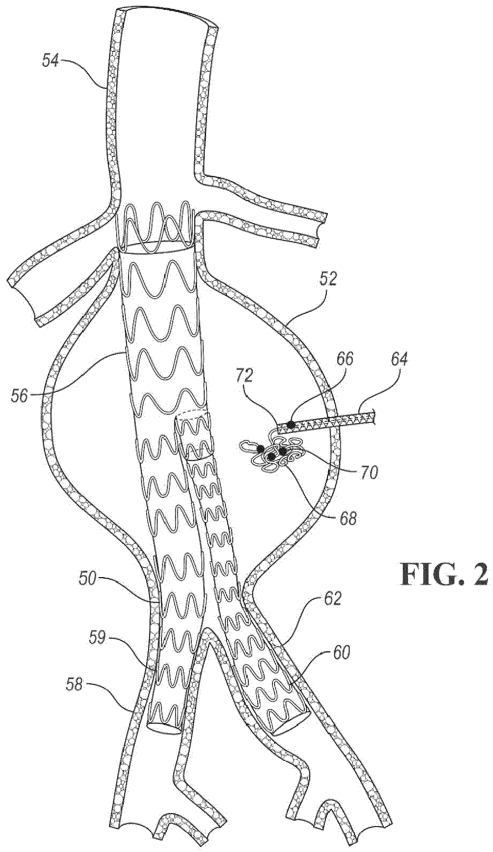

Figure 2 is a side view of stent graft 50 in a radially expanded state such that stent graft 50 is deployed within aneurysm 52 to bridge a damaged portion of aorta 54. As shown in Figure 2, stent graft 50 is a branched stent graft including main body 56 extending into leg 59 extending into first iliac artery 58 and leg 60 extending into second iliac artery 62. In other embodiments, the stent graft may only include a main body without branching or it may include additional or different branches. Figure 2 also depicts ancillary device 64 introduced into the sac of aneurysm 52. Ancillary device 64 may be introduced into the sac of aneurysm 52 using a variety of procedures, including, without limitation, (1) translumbar puncture, (2) reintervention to obtain vascular access, (3) venous side puncture into an artery, and (4) intercostal vascular access through superior mesenteric artery (SMA). Ancillary device 64 integrates pressure sensor 66. In other embodiments, ancillary device 64 integrates two sensors (e.g., distal and proximal pressure sensors) to output pressure sensor data usable to determine a pressure gradient between the distal and proximal locations of the distal and proximal pressure sensors, respectively. The second sensor may be integrated by ancillary device 64 or may be independent of ancillary device 64 (e.g., a sensor located at a different access site or by using a blood pressure cuff). The second sensor may be configured to output (e.g., continuously output) pressure data indicative of systemic blood pressure.

Ancillary device 64 may be configured to determine whether an endoleak is present or if an endoleak's repair (e.g., after the index procedure) has been successful. For example, this determination using ancillary device 64 may be beneficial to detect a type #2 endoleak or after repair of a type #2 endoleak caused by pressurization of the sac from blood perfusing through a vessel in communication with the sac. Pressure sensor 66 may continuously output pressure data indicative of the pressure in the sac. Pressure reducing agents (e.g., embolic coils 68, liquid embolic material 70, and/or endoanchors) are introduced in the sac (or other abnormality cavity) through distal end 72 of ancillary device 64 (shown as a catheter) until a reduced pressure output by pressure sensor 66 or a pressure gradient determined by pressure data output by two sensors reaches a threshold pressure reduction (e.g., an attenuation of blood pressure in the aneurysm sac relative to systemic pressure is appreciated).

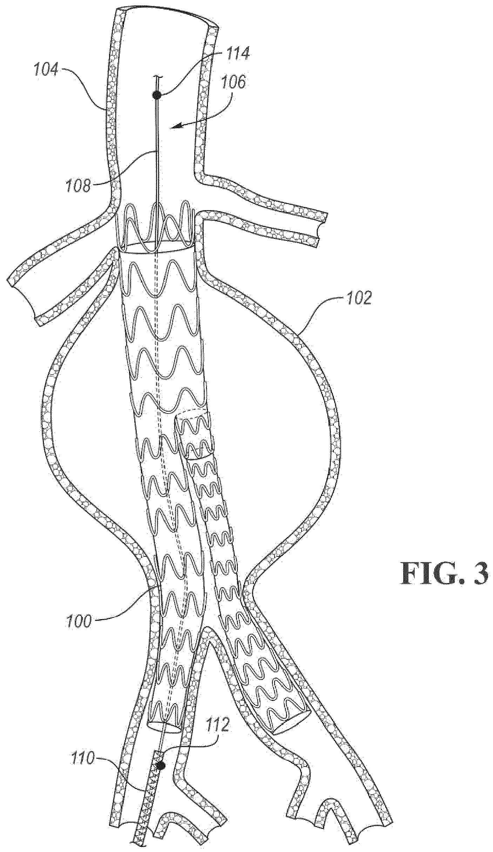

Figure 3 is a side view of stent graft 100 in a radially expanded state such that stent graft 100 is deployed within aneurysm 102 to bridge a damaged portion of aorta 104. Figure 3 depicts ancillary device 106 deployed within deployed stent graft 100. Ancillary device 106 includes guidewire 108 and catheter 110 configured to move along guidewire 108. Ancillary device 106 includes distal and proximal pressure sensors 112 and 114. Proximal pressure sensor 114 is integrated into guidewire 108. Distal pressure sensor 112 is integrated into catheter 110. As shown in Figure 3, distal pressure sensor 112 is located distal a distal end of deployed stent graft 100 and proximal pressure sensor 114 is located proximal a proximal end of deployed stent graft 100.

Ancillary device 106 may be configured to detect the presence of a partial occlusion, e.g., due to a kink in deployed stent graft 100 or an unresolved vascular pathology resulting in a pressure gradient across the implant. Proximal pressure sensor 114 is advanced through deployed stent graft 100 and is configured to output pressure data indicative of systemic blood pressure while catheter 110 carrying distal pressure sensor 112 is positioned distal deployed stent graft 110. A contralateral limb may be measured in a similar way. A pressure gradient between the two sensors differing by at least any of the following values or in a range of any two of the following values: 10, 15, 20, and 25% may be clinically significant, prompting a physician to take action to resolve the flow disturbance prior to completion of the case, thereby lowering the rate of stent graft occlusion and reducing the number of secondary interventions. The action may alleviate the flow disturbance over a relatively short period of time (e.g., immediately or under a minute). The pressure data output from distal and proximal sensors 112 and 114 may be used by a controller to determine that the pressure gradient no longer exists in real time. This embodiment may be able to detect flow disturbances within deployed stent grafts that are not detectable using two-dimensional (2D) visual output from fluoroscopy.

The locations of distal and/or proximal pressure sensors 112 and 114 on guidewire 108 and/or catheter 110, respectively, may be adjusted provided that a distance is maintained between distal and proximal sensors 112 and 114 so that pressure data output from the sensors may be used to determine a pressure gradient between the locations. Similar to above, the pressure gradient may be displayed continuously, intermittently, or instantaneously upon request.

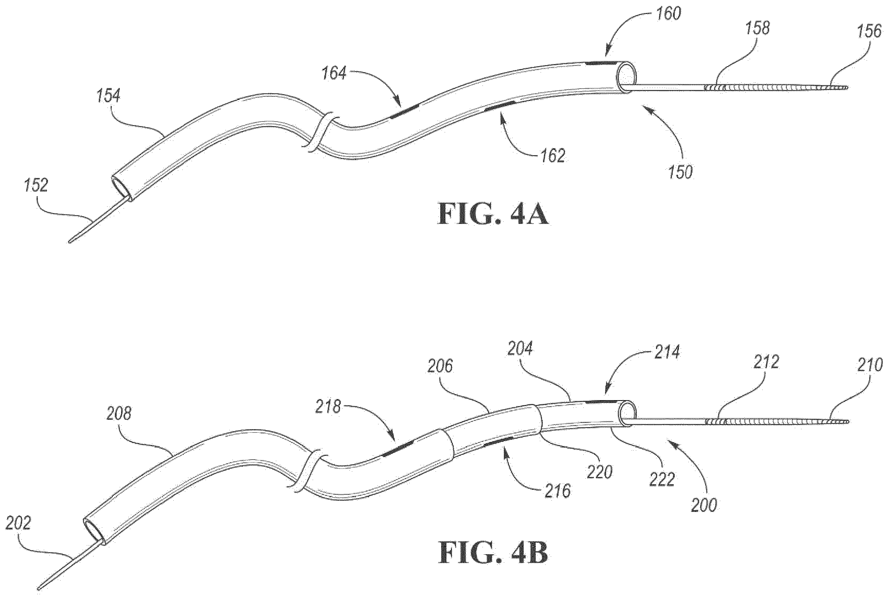

Figure 4A is a distal side view of assembled ancillary devices 150 including first and second ancillary device components guidewire 152 and sheath 154. As shown in Figure 4A, first ancillary device component 152 is a guidewire integrating pressure sensors 156 and 158 at a distal portion thereof. First ancillary device component 152 may include one or more pressure sensors evenly or unevenly spaced along a distal portion thereof. The second ancillary device component may be a catheter, a sheath, or a balloon. As shown in Figure 4A, second ancillary device component 154 is a catheter integrating pressure sensors 160, 162, and 164. Second ancillary device component 154 may include one or more pressure sensors evenly or unevenly spaced along a distal portion thereof. The use of multiple sensors provides greater pressure data for monitoring a pressure gradient's presence or absence in connection with a potential endoleak. The first ancillary device component may be an arterial or venous line (e.g., a fluid filled catheter attached to a pressure transducer) used to monitor patient vitals during the procedure, such as those used by an interventionalist. This line may be used by the implanting physician to measure sac pressures through a guide catheter. The second ancillary device component may be a catheter movable relative the arterial or venous line. In another embodiment, a pressure monitoring system separate from existing, ancillary devices can be used.

Figure 4B is a distal side view of ancillary devices 200 including guidewire 202 and first, second and third nested catheters 204, 206, and 208 moveable relative each other and guidewire 202. Guidewire 202 includes pressure sensors 210 and 212 at a distal portion thereof. First nested catheter 204 integrates pressure sensor 214, second nested catheter 206 integrates pressure sensor 216, and third nested catheter 208 integrates pressure sensor 218. The outer diameter of first nested catheter 204 is less than or equal to the inner diameter of second nested catheter 206, which has an outer diameter less than or equal to the inner diameter of third nested catheter 208, thereby permitting relative movement of the nested catheters to each other to change the relative position pressure sensors 214, 216, and 218 relative to each other, which allows for convenient adjustments when outputting location-based pressure data to determine the presence or absence of endoleaks. During deployment of the nested catheters, distal end 220 of second nested catheter 206 may be located proximal distal end portion 220 of first nested catheter 204. As shown in Figure 4B, distal end portion 220 integrates pressure sensor 214. Each of the sensors outputs pressure data indicative of pressure over time at the location of the sensor in the vasculature of the patient. This pressure data may be utilized to determine a pressure gradient indicative of the presence or absence of endoleaks.

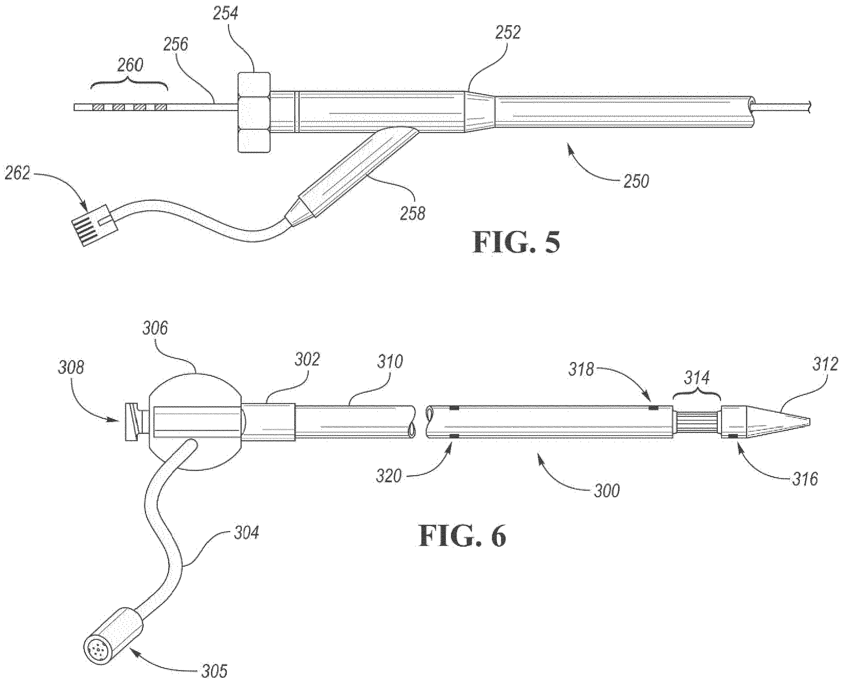

Figure 5 is a proximal side view of assembled ancillary devices 150 shown in Figure 4A. Proximal body 250 includes handle 252 and hemostasis valve 254 and includes extension arm 258 for electrical conductors attached to electrical connector 262. Electrical connector 262 is configured to electrically connect the one or more sensors integrated with the sheath 154. Electrical connector 262 interfaces with a pressure reading apparatus (not shown). Guidewire 256 extends through proximal handle body 250. Guidewire 256 includes contacts 260 configured to electrically connect one or more sensors integrated within guidewire 256 which in turn are interfaced with external slip on type connector (not shown) that interfaces with a pressure reading apparatus (not shown). Contacts 260 are configured to interface with connector 262 to output the pressure data to a controller (not shown). Proximal body 250 and integral handle 252, hemostasis valve 254, and extension arm 258 for electrical conductors attached to electric connector 262 is a typical proximal configuration for first, second and third nested catheters 204, 206, and 208 shown in Figure 4B.

Figure 6 is a side view of ancillary imaging device delivery system 300. Ancillary imaging device delivery system 300 includes handle 302 and connector housing 304 terminating at connector 305. Luer 306 is configured to connect handle 302 and connector housing 304. Guidewire lumen 308 is disposed at the proximal end of handle 302. Catheter 310 extends from housing 304. Ancillary imaging device delivery system 300 also includes nose cone 312 at a distal end thereof and imaging element 314 extending between catheter 310 and nose cone 312. Nose cone 312 integrates distal pressure sensor 316. Catheter 310 includes first proximal pressure sensor 318 and optionally second proximal pressure sensor 320. Each of the sensors outputs pressure data indicative of pressure over time at the location of the sensor in the vasculature of the patient. This pressure data may be utilized to determine a pressure gradient indicative of the presence or absence of endoleaks.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

Further disclosed herein is the subject-matter of the following clauses:
1. A method for determining a pressure gradient between proximal and distal locations within a vasculature of a patient, the method comprising:
   deploying an ancillary device within the vasculature, the ancillary device integrating a proximal sensor at the proximal location and a distal sensor at the distal location;
   deploying, via a stent graft delivery system, a stent graft within the vasculature to obtain a deployed stent graft, the ancillary device independent from the stent graft delivery system and the stent graft;
   outputting proximal pressure data from the proximal sensor and indicative of pressure at the proximal location and distal pressure data from the distal sensor and indicative of pressure at the distal location; and
   determining the pressure gradient using the proximal and distal pressure data.
2. The method of clause 1, wherein the ancillary device is situated outside the deployed stent graft while the outputting step is performed.
3. The method of clause 1 or of any of clauses 1-2, wherein the outputting step is performed during a monitoring period relative to a completion of the second deploying step.
4. The method of clause 1 or of any of clauses 1-3, wherein the ancillary device includes an ancillary guidewire integrating the proximal sensor and an ancillary catheter integrating the distal sensor, and the ancillary catheter is movable relative to the ancillary guidewire.
5. The method of clause 4, wherein the first deploying step including moving the ancillary catheter relative to the ancillary guidewire such that the proximal sensor reaches the proximal location.
6. The method of clause 1 or of any of clauses 1-5, wherein the first deploying step occurs before the second deploying step.
7. The method of clause 1 or of any of clauses 1-6, wherein the distal location is located within an abnormality cavity formed between an abnormality wall and the deployed stent graft within the abnormality and the proximal location is located outside the abnormality cavity.
8. The method of clause 7, further comprising introducing a pressure reducing agent into the abnormality cavity through a distal end of the ancillary device until the pressure gradient reaches a threshold pressure gradient.
9. The method of clause 1 or of any of clauses 1-8, wherein the first deploying step includes deploying the ancillary device within the deployed stent graft.
10. The method of clause 9, wherein the proximal location is located proximal a proximal end of the deployed stent graft and the distal location is located distal a distal end of the deployed stent graft.
11. The method of clause 1 or of any of clauses 1-10, wherein the outputting step includes simultaneously outputting the proximal pressure data from the proximal sensor and the distal pressure data from the distal sensor.
12. A method for determining a pressure gradient between proximal and distal locations within a vasculature of a patient, the method comprising:
   deploying a guidewire within the vasculature;
   deploying first and second nested catheters over the guidewire within the vasculature, the first nested catheter integrating a distal sensor at the distal location, and the second nested catheter integrating a proximal sensor at the proximal location;
   deploying a stent graft within the vasculature to obtain a deployed stent graft;
   outputting proximal pressure data from the proximal sensor and indicative of pressure at the proximal location and distal pressure data from the distal sensor and indicative of pressure at the distal location; and
   determining the pressure gradient using the proximal and distal pressure data.
13. The method of clause 12, wherein the first nested catheter has an outer diameter less than or equal to an inner diameter of the second nested catheter, and the second deploying step including first deploying the first nested catheter and secondly deploying the second nested catheter.
14. The method of clause 13, wherein a distal end of the second nested catheter is located proximal a distal end portion of the first nested catheter.
15. The method of clause 14, wherein the distal end portion of the first nested catheter integrates the distal sensor.
16. An endovascular pressure sensing device for outputting pressure data indicative of a pressure gradient between proximal and distal locations within a vasculature of a patient, the endovascular pressure sensing device comprising:
   an ancillary device deployable within the vasculature relative to a stent graft deployable within the vasculature, the ancillary device independent from the stent graft; and
   proximal and distal sensors, the ancillary device configured to integrate the proximal sensor at the proximal location and the distal sensor at the distal location, the proximal sensor configured to output proximal pressure data indicative of pressure at the proximal location and the distal sensor configured to output distal pressure data indicative of pressure at the distal location.
17. The endovascular pressure sensing device of clause 16, wherein the ancillary device includes a first ancillary device component configured to integrate the distal sensor and a second ancillary device component configured to integrate the proximal sensor and movable relative to the first ancillary device.
18. The endovascular pressure sensing device of clause 17, wherein the second ancillary device component is an ancillary guidewire and the first ancillary device component is selected from the group consisting of: a catheter, a sheath, and a balloon.
19. The endovascular pressure sensing device of clause 18, further comprising an electrical connector configured to output the proximal and/or distal pressure data and the ancillary guidewire includes one or more electrical contacts configured to electrically connect the distal sensor to the electrical connector.
20. The endovascular pressure sensing device of clause 17, wherein one of the first or second ancillary device components is an arterial or venous guidewire and the other of the first or second ancillary device components is a catheter.
21. A system configured for determining a pressure gradient between proximal and distal locations within a vasculature of a patient, the system comprising:
   an ancillary device configured for deploying within the vasculature, the ancillary device integrating a proximal sensor at the proximal location and a distal sensor at the distal location;
   a stent graft configured for deploying, via a stent graft delivery system, within the vasculature to obtain a deployed stent graft, the ancillary device independent from the stent graft delivery system and the stent graft;
   the system being further configured outputting proximal pressure data from the proximal sensor and indicative of pressure at the proximal location and distal pressure data from the distal sensor and indicative of pressure at the distal location; and
   the system being configured for determining the pressure gradient using the proximal and distal pressure data.
22. The system of clause 21, wherein the system is configured such that the ancillary device is situated outside the deployed stent graft while the outputting step is performed.
23. The system of any of clauses 21-22, wherein the system is configured such that the outputting step is performed during a monitoring period relative to a completion of the second deploying step.
24. The system of any of clauses 21-23, wherein the ancillary device includes an ancillary guidewire integrating the proximal sensor and an ancillary catheter integrating the distal sensor, and the ancillary catheter is movable relative to the ancillary guidewire.
25. The system of clause 24, wherein the system is configured such that the first deploying step including moving the ancillary catheter relative to the ancillary guidewire such that the proximal sensor reaches the proximal location.
26. The system of any of clauses 21-25, wherein the system is configured such that the first deploying step occurs before the second deploying step.
27. The system of any of clauses 21-26, wherein the system is configured such that the distal location is located within an abnormality cavity formed between an abnormality wall and the deployed stent graft within the abnormality and the proximal location is located outside the abnormality cavity.
28. The system of clause 27, wherein the system further comprises a pressure reducing agent configured for introducing into the abnormality cavity through a distal end of the ancillary device until the pressure gradient reaches a threshold pressure gradient.
29. The system of any of clauses 21-28, wherein the system is configured such that the first deploying step includes deploying the ancillary device within the deployed stent graft.
30. The system of clause 29, wherein the system is configured such that the proximal location is located proximal a proximal end of the deployed stent graft and the distal location is located distal a distal end of the deployed stent graft.
31. The system of any of clauses 21-30, wherein the system is configured such that the outputting step includes simultaneously outputting the proximal pressure data from the proximal sensor and the distal pressure data from the distal sensor.
32. A system for determining a pressure gradient between proximal and distal locations within a vasculature of a patient, the system comprising:
   a guidewire configured for deploying within the vasculature;
   first and second nested catheters configured for deploying over the guidewire within the vasculature, the first nested catheter integrating a distal sensor at the distal location, and the second nested catheter integrating a proximal sensor at the proximal location;
   a stent graft configured for deploying within the vasculature to obtain a deployed stent graft;
   wherein the system is configured for outputting proximal pressure data from the proximal sensor and indicative of pressure at the proximal location and distal pressure data from the distal sensor and indicative of pressure at the distal location; and
   wherein the system is configured for determining the pressure gradient using the proximal and distal pressure data.
33. The system of clause 32, wherein the first nested catheter has an outer diameter less than or equal to an inner diameter of the second nested catheter, and wherein the system is configured such that the second deploying step includes first deploying the first nested catheter and secondly deploying the second nested catheter.
34. The system of clause 33, wherein the system is configured such that a distal end of the second nested catheter is located proximal a distal end portion of the first nested catheter.
35. The system of clause 34, wherein the distal end portion of the first nested catheter integrates the distal sensor.

## Claims

1. An endovascular pressure sensing device for outputting pressure data indicative of a pressure gradient between proximal and distal locations within a vasculature of a patient, the endovascular pressure sensing device comprising:
an ancillary device deployable within the vasculature relative to a stent graft deployable within the vasculature, the ancillary device independent from the stent graft; and
proximal and distal sensors, the ancillary device configured to integrate the proximal sensor at the proximal location and the distal sensor at the distal location, the proximal sensor configured to output proximal pressure data indicative of pressure at the proximal location and the distal sensor configured to output distal pressure data indicative of pressure at the distal location.

2. The endovascular pressure sensing device of claim 1, wherein the ancillary device includes a first ancillary device component configured to integrate the distal sensor and a second ancillary device component configured to integrate the proximal sensor and movable relative to the first ancillary device.

3. The endovascular pressure sensing device of claim 2, wherein the second ancillary device component is an ancillary guidewire and the first ancillary device component is selected from the group consisting of: a catheter, a sheath, and a balloon.

4. The endovascular pressure sensing device of claim 3, further comprising an electrical connector configured to output the proximal and/or distal pressure data and the ancillary guidewire includes one or more electrical contacts configured to electrically connect the distal sensor to the electrical connector.

5. The endovascular pressure sensing device of claim 2, wherein one of the first or second ancillary device components is an arterial or venous guidewire and the other of the first or second ancillary device components is a catheter.

6. A system configured for determining a pressure gradient between proximal and distal locations within a vasculature of a patient, the system comprising:
an ancillary device configured for deploying within the vasculature, the ancillary device integrating a proximal sensor at the proximal location and a distal sensor at the distal location;
a stent graft configured for deploying, via a stent graft delivery system, within the vasculature to obtain a deployed stent graft, the ancillary device independent from the stent graft delivery system and the stent graft;
the system being further configured outputting proximal pressure data from the proximal sensor and indicative of pressure at the proximal location and distal pressure data from the distal sensor and indicative of pressure at the distal location; and
the system being configured for determining the pressure gradient using the proximal and distal pressure data.

7. The system of claim 6, wherein the system is configured such that the ancillary device is situated outside the deployed stent graft while the outputting step is performed.

8. The system of any of claims 6-7, wherein the system is configured such that the outputting step is performed during a monitoring period relative to a completion of the second deploying step.

9. The system of any of claims 6-8, wherein the ancillary device includes an ancillary guidewire integrating the proximal sensor and an ancillary catheter integrating the distal sensor, and the ancillary catheter is movable relative to the ancillary guidewire, wherein typically the system is configured such that the first deploying step including moving the ancillary catheter relative to the ancillary guidewire such that the proximal sensor reaches the proximal location.

10. The system of any of claims 6-9, wherein the system is configured such that the first deploying step occurs before the second deploying step.

11. The system of any of claims 6-10, wherein the system is configured such that the distal location is located within an abnormality cavity formed between an abnormality wall and the deployed stent graft within the abnormality and the proximal location is located outside the abnormality cavity, wherein the system typically further comprises a pressure reducing agent configured for introducing into the abnormality cavity through a distal end of the ancillary device until the pressure gradient reaches a threshold pressure gradient.

12. The system of any of claims 6-11, wherein the system is configured such that the first deploying step includes deploying the ancillary device within the deployed stent graft, wherein typically the system is configured such that the proximal location is located proximal a proximal end of the deployed stent graft and the distal location is located distal a distal end of the deployed stent graft.

13. The system of any of claims 6-12, wherein the system is configured such that the outputting step includes simultaneously outputting the proximal pressure data from the proximal sensor and the distal pressure data from the distal sensor.

14. A system for determining a pressure gradient between proximal and distal locations within a vasculature of a patient, the system comprising:
a guidewire configured for deploying within the vasculature;
first and second nested catheters configured for deploying over the guidewire within the vasculature, the first nested catheter integrating a distal sensor at the distal location, and the second nested catheter integrating a proximal sensor at the proximal location;
a stent graft configured for deploying within the vasculature to obtain a deployed stent graft;
wherein the system is configured for outputting proximal pressure data from the proximal sensor and indicative of pressure at the proximal location and distal pressure data from the distal sensor and indicative of pressure at the distal location; and
wherein the system is configured for determining the pressure gradient using the proximal and distal pressure data.

15. The system of claim 14, wherein the first nested catheter has an outer diameter less than or equal to an inner diameter of the second nested catheter, and wherein the system is configured such that the second deploying step includes first deploying the first nested catheter and secondly deploying the second nested catheter, wherein the system is configured such that a distal end of the second nested catheter is located proximal a distal end portion of the first nested catheter, wherein typically the distal end portion of the first nested catheter integrates the distal sensor.
